# EUROPEAN PATENT APPLICATION

(11) **EP 4 802 987 A1**
(43) Date of publication of application: **09.09.2026**
(21) Application number: 26162082.7
(22) Date of filing: 03.03.2026
(51) Int. Cl.: A61B 1/00, A61B 1/313, A61B 5/00, A61B 8/00

(54) **MEDICAL DEVICE AND OPERATION METHOD THEREOF**

(30) Priority: 04.03.2025 JP 2025033501
(71) Applicant: FUJIFILM Corporation, Tokyo 106-8620 (JP)
(72) Inventor: MACHII, Yusuke, Tokyo, 106-8620 (JP)
(74) Representative: Dehns Germany Partnerschaft mbB

(57) **Abstract**

The present disclosure provides a medical device (12) and an operation method thereof that can prevent, even in a case where a video of a superimposition target (OV) to be superimposed is superimposed on a camera video such as a laparoscopic video (30), an incident in a superimposed region from being overlooked.

A camera video acquisition unit (20) acquires a laparoscopic video (30). A video acquisition unit for superimposition (21) acquires a video of a superimposition target (OV) on the laparoscopic video (30). A detection unit (23) detects a change in a superimposition region in which the video of the superimposition target (OV) to be superimposed is superimposed on the laparoscopic video (30), and outputs any of notification necessity information according to a result of the detection, a superimposition parameter for controlling display of the superimposition region, or an incident occurrence region.

## Description

### BACKGROUND OF THE INVENTION

### 1. Field of the Invention

The present disclosure relates to a medical device used for observation or surgery in an abdominal cavity, such as in laparoscopic procedures, and an operation method thereof.

### 2. Description of the Related Art

In order to safely perform surgery in laparoscopic procedures, it is important to consider intra-organ information such as blood vessels and lesion locations. As means for referring to the intra-organ information, there are intraoperative ultrasound videos, preoperative three-dimensional (3D) data, and the like. In a case of referring to the ultrasound video, the preoperative 3D data, and the like, the preoperative 3D data is superimposed and displayed on a laparoscopic video, thereby eliminating the need for a line-of-sight shift from the laparoscopic video and reducing operator's cognitive load (for example, JP2007-7041A).

### SUMMARY OF THE INVENTION

As described above, in a case of superimposing and displaying a video of a superimposition target, such as the ultrasound video or the preoperative 3D data, on a camera video such as the laparoscopic video, the superimposed region is less visible to a user, and may be invisible, so that an incident such as bleeding or bile leakage may be overlooked.

An object of the present disclosure is to provide a medical device and an operation method thereof that can prevent, even in a case where a video of a superimposition target is superimposed on a camera video such as a laparoscopic video, an incident in a superimposed region from being overlooked.

According to the present disclosure, there is provided a medical device comprising: a processor, in which the processor acquires a camera video, acquires a video of a superimposition target to be superimposed on the camera video, and detects a change in a superimposition region in which the video of the superimposition target is superimposed on the camera video, and outputs any of notification necessity information according to a result of the detection, a superimposition parameter for controlling display of the superimposition region, or an incident occurrence region.

It is preferable that, in a case where the processor outputs the notification necessity information, in a case where the notification necessity information indicates that notification is unnecessary, the processor perform a single-screen display of a main screen that displays the camera video on which the video of the superimposition target is superimposed, and, in a case where the notification necessity information indicates that notification is necessary, the processor perform a two-screen display of the main screen and a sub-screen that performs display related to the superimposition region. It is preferable that the processor perform either increasing a transparency of the superimposition region or temporarily cancelling superimposition of the superimposition region by increasing or decreasing the superimposition parameter according to the result of the detection. It is preferable that, in a case where the processor outputs the incident occurrence region, the processor perform either increasing a transparency of the incident occurrence region in the superimposition region or temporarily cancelling superimposition of the incident occurrence region in the superimposition region.

It is preferable that the processor detect a change in a pixel value of the superimposition region. It is preferable that the processor detect an outflow of a body fluid in the superimposition region. It is preferable that, in a case where the processor detects at least one of bleeding or bile leakage based on the change in the pixel value, the processor output the notification necessity information indicating that notification is necessary. It is preferable that, in a case where the processor detects at least one of bleeding or bile leakage based on a detection result of the outflow of the body fluid, the processor output the notification necessity information indicating that notification is necessary.

It is preferable that the video of the superimposition target be at least one of an ultrasound video, or a video of an anatomical structure or a lesion in preoperative 3D data. It is preferable that a superimposition target be at least one of a liver, a kidney, a spleen, a pancreas, a uterus, a nerve, a lung, a bronchus, an intracranial blood vessel, a prostate, or an organ that emits light due to a fluorescent dye.

According to the present disclosure, there is provided an operation method of a medical device including a processor, the operation method comprising: a step of, via the processor, acquiring a camera video; a step of, via the processor, acquiring a video of a superimposition target superimposed on the camera video; and a step of, via the processor, detecting a change in a superimposition region in which the video of the superimposition target is superimposed on the camera video, and outputting any of notification necessity information according to a result of the detection, a superimposition parameter for controlling display of the superimposition region, or an incident occurrence region.

According to the present disclosure, it is possible to prevent, even in a case where a video of a superimposition target is superimposed on a camera video such as a laparoscopic video, an incident in a superimposed region from being overlooked.

### BRIEF DESCRIPTION OF THE DRAWINGS

FIG. 1 is a schematic diagram of a medical system.
FIG. 2A is an image diagram of a display that displays a laparoscopic video on which an ultrasound video is superimposed, and FIG. 2B is an image diagram of a display that displays a laparoscopic video on which an anatomical structure in preoperative 3D data is superimposed.
FIG. 3 is an image diagram of a display in a case where notification necessity information indicates that notification is unnecessary.

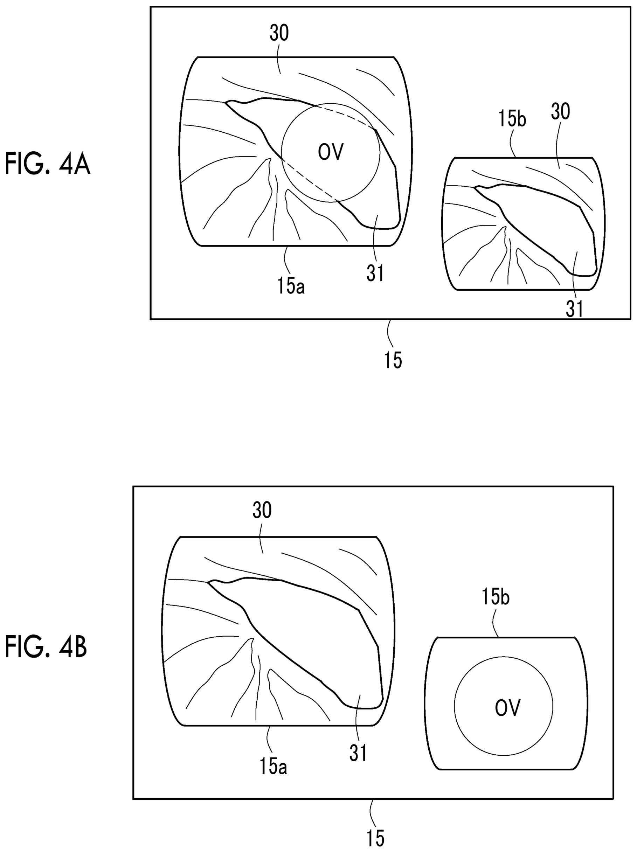

FIG. 4A is an explanatory diagram showing a case where a laparoscopic video on which a video of a superimposition target is not superimposed is displayed on a sub-screen in a case of displaying contents of a main screen and the sub-screen in a case where the notification necessity information indicates that notification is necessary, and FIG. 4B is an explanatory diagram showing a case where a video of a superimposition target is displayed on a sub-screen in a case of displaying contents of a main screen and the sub-screen in a case where the notification necessity information indicates that notification is necessary.
FIG. 5A is an explanatory diagram showing a case where a superimposition parameter is not output, and FIG. 5B is an explanatory diagram showing a case where the superimposition parameter is output.
FIG. 6A is an explanatory diagram showing a case where an incident occurrence region is not output, and FIG. 6B is an explanatory diagram showing a case where the incident occurrence region is output.
FIG. 7A is a block diagram showing a detection unit configured with a superimposition region detection unit and an incident detection unit, and FIG. 7B is a block diagram showing a detection unit configured with an incident detection unit and a notification determination unit.
FIG. 8 is a flowchart showing a series of flows of outputting the notification necessity information according to a result of a change in a superimposition target.

### DESCRIPTION OF THE PREFERRED EMBODIMENTS

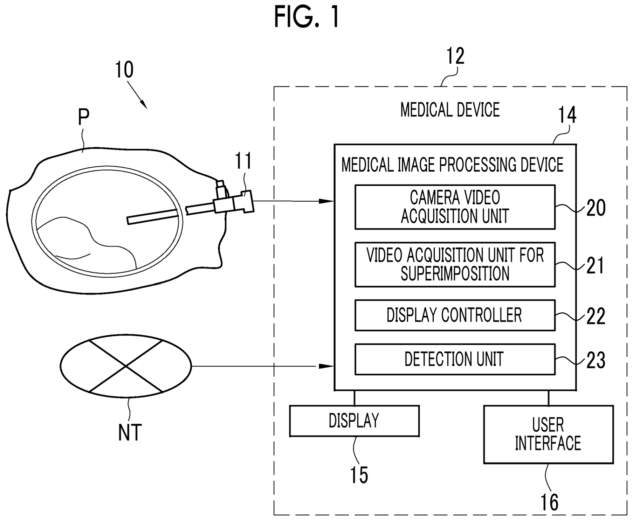

As shown in FIG. 1, a medical system 10 comprises a laparoscope 11 and a medical device 12. The laparoscope 11 captures an image of an inside of a body of a patient P and transmits a laparoscopic video obtained by the capturing to the medical device 12. The laparoscope 11 is also connected to a light source device (not shown), and illumination light from the light source device is supplied to the laparoscope 11.

The medical device 12 comprises a medical image processing device 14 configured by a computer such as a server, a display 15, and a user interface 16. In addition, the medical image processing device 14 is connected to a network NT. A picture archiving and communication system (PACS) or the like is connected to the network NT, and various image data and the like from the PACS are incorporated into the medical image processing device 14 via the network NT.

In the medical image processing device 14, a program for executing various types of processing is stored in a program memory (not shown). A central controller (not shown) configured by a processor executes the program in the program memory, whereby the medical image processing device 14 implements functions of a camera video acquisition unit 20, a video acquisition unit for superimposition 21, a display controller 22, and a detection unit 23.

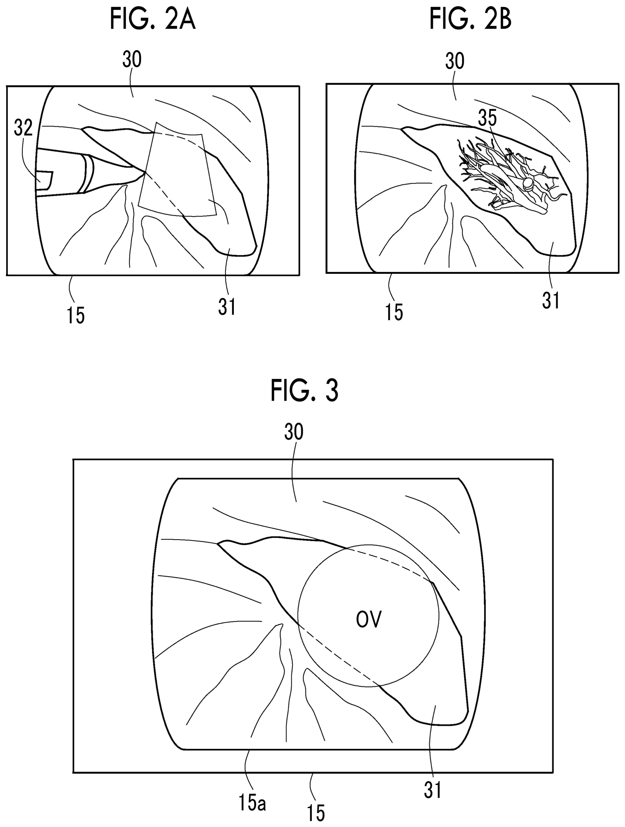

The camera video acquisition unit 20 acquires a camera video. In the present embodiment, the laparoscope 11 captures the camera video. The video acquisition unit for superimposition 21 acquires a video of a superimposition target to be superimposed on the camera video. It is preferable that the video of the superimposition target be at least one of an ultrasound video, or a video of an anatomical structure or a lesion in preoperative 3D data. It is preferable that the ultrasound video be an ultrasound video obtained by an ultrasound probe (see FIG. 2A) used in an abdominal cavity of the patient P, such as the laparoscope 11. It is preferable that the preoperative 3D data be a 3D organ model obtained before observation with the laparoscope 11, and be a radiation image such as an X-ray image or a CT image, or a 3D image extracted from an MRI. It is preferable that a superimposition target be at least one of a liver, a kidney, a spleen, a pancreas, a uterus, a nerve, a lung, a bronchus, an intracranial blood vessel, a prostate, or an organ that emits light due to a fluorescent dye (for example, Indo Cyanine Green (ICG)). It is preferable that the camera video be a color video, and various medical videos other than the laparoscopic video may be used.

The display controller 22 controls the display of the display 15. In the present embodiment, the display controller 22 causes the display 15 to display a laparoscopic video on which the video of the superimposition target is superimposed. Specifically, in a case where the target organ is the liver and the video of the superimposition target is the ultrasound video obtained by the ultrasound probe in the abdominal cavity, as shown in FIG. 2A, an ultrasound video 33 obtained by an ultrasound probe 32 is superimposed and displayed on a region of a liver 31 in a laparoscopic video 30. In addition, in a case where the target organ is the liver and the video of the superimposition target is the anatomical structure of the preoperative 3D data, as shown in FIG. 2B, a vascular structure 35 inside the liver is superimposed and displayed on the region of the liver 31 in the laparoscopic video 30 as the anatomical structure of the preoperative 3D data. The structure to be superimposed on the laparoscopic video is not limited to the anatomical structure of the liver, and may be a structure of another organ.

As described above, in the medical device 12 of the present embodiment, by superimposing the video of the superimposition target on the laparoscopic video, a user can understand internal information of the superimposition target without performing a line-of-sight shift. On the other hand, a superimposition region in which the video of the superimposition target is superimposed on the laparoscopic video is less visible to the user or becomes invisible, so that an incident such as bleeding or bile leakage may be overlooked. With respect to this, in the present embodiment, the detection unit 23 detects a change in the superimposition region, and outputs any of notification necessity information according to a result of the detection, a superimposition parameter for controlling display of the superimposition region, or an incident occurrence region. The superimposition region includes, for example, a mask representing region information or coordinate information of an outer edge of the region.

The display controller 22 controls the display of the display 15 based on the notification necessity information. Specifically, in a case where the notification necessity information indicates that notification is unnecessary, the display controller 22 performs a single-screen display of a main screen that displays the camera video on which the video of the superimposition target is superimposed, and, in a case where the notification necessity information indicates that notification is necessary, the display controller 22 performs a two-screen display of the main screen and a sub-screen that performs display related to the superimposition region. For example, as shown in FIG. 3, in a case where the notification necessity information indicates that notification is unnecessary, a single-screen display is performed in which the laparoscopic video 30 on which a video of a superimposition target OV is superimposed is displayed on a main screen 15a provided on the entire display 15.

On the other hand, as shown in FIG. 4A, in a case where the notification necessity information indicates that notification is necessary, a two-screen display of the main screen 15a and a sub-screen 15b is displayed on the display 15. Then, the laparoscopic video 30 on which the video of the superimposition target OV is superimposed is displayed on the main screen 15a. On the other hand, the laparoscopic video 30 on which the superimposition of the video of the superimposition target is hidden is displayed on the sub-screen 15b. As a result, the state of the change in the superimposition region can be understood on the sub-screen 15b while the video of the superimposition target is observed on the main screen 15a.

In addition, as shown in FIG. 4B, in a case where the notification necessity information indicates that notification is necessary, on the main screen 15a, the superimposition of the video of the superimposition target OV may be cancelled, and the laparoscopic video 30 on which the video of the superimposition target OV is not superimposed may be displayed. On the other hand, the video of the superimposition target OV is displayed on the sub-screen 15b. As a result, the state of the change in the superimposition region can be understood on the main screen 15a, and the video of the superimposition target can be checked on the sub-screen 15b.

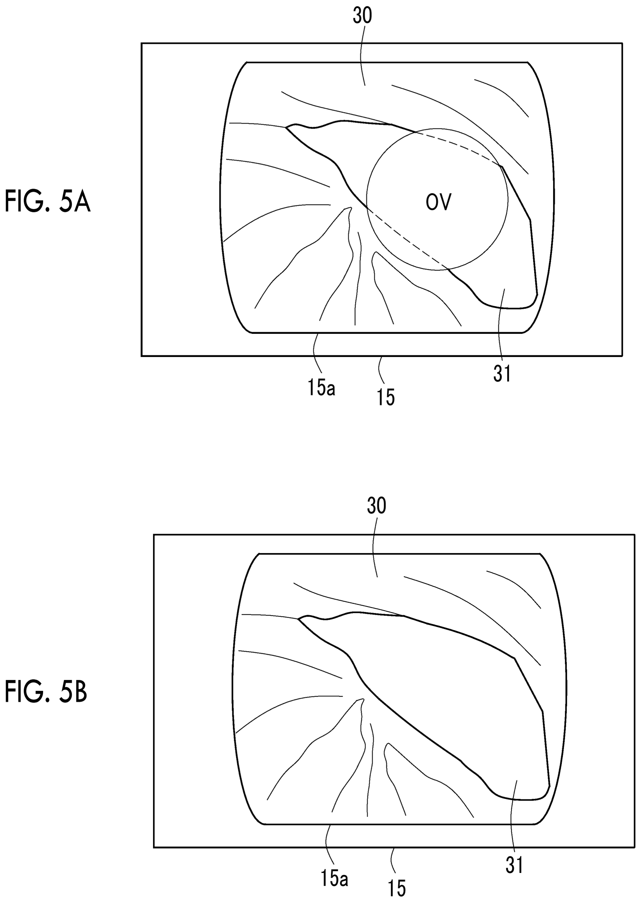

The display controller 22 may perform either increasing a transparency of the superimposition region or temporarily cancelling superimposition of the superimposition region by increasing or decreasing the superimposition parameter according to the detection result of the change in the superimposition region. For example, as shown in FIG. 5A, in a situation in which the laparoscopic video 30 on which the video of the superimposition target OV is superimposed is displayed on the main screen 15a, in a case where the display controller 22 receives the superimposition parameter, the superimposition of the video of the superimposition target OV is cancelled, and only the laparoscopic video 30 is displayed on the main screen 15a, as shown in FIG. 5B. It is preferable that the transparency of the superimposition region be set to 0% in a case where an incident has not occurred, and the transparency of the superimposition region be set to 100% in a case where an incident has occurred. In addition, it is preferable that the superimposition of the superimposition region be resumed in a case where a superimposition button (not shown) is pressed again or in a case where the occurrence of the incident is not detected.

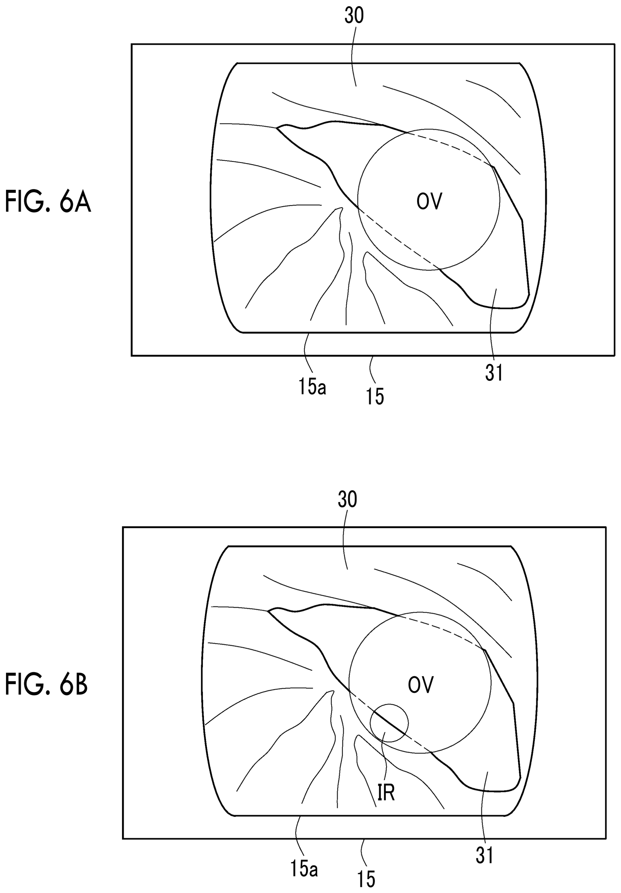

In a case where the incident occurrence region is output, the display controller 22 may perform either increasing the transparency of the incident occurrence region in the superimposition region or temporarily cancelling the superimposition of the video of the superimposition target in the incident occurrence region in the superimposition region. For example, as shown in FIG. 6A, in a situation in which the laparoscopic video 30 on which the video of the superimposition target OV is superimposed is displayed on the main screen 15a, in a case where the display controller 22 receives the incident occurrence region, the superimposition of the video of the superimposition target OV in the incident occurrence region IR in the superimposition region is temporarily cancelled, as shown in FIG. 6B. In a case where the superimposition of the superimposition region is resumed, the same applies as in the above case.

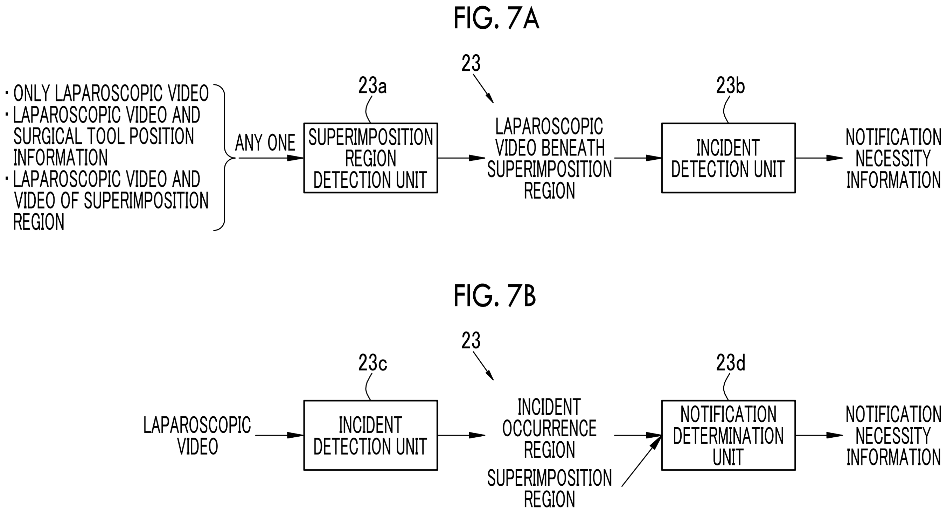

As shown in FIG. 7A, the detection unit 23 may be divided into two units of a superimposition region detection unit 23a and an incident detection unit 23b. In this case, any of the laparoscopic video only, the laparoscopic video and surgical tool position information, or both the laparoscopic video and the video of the superimposition region is input to the superimposition region detection unit 23a. The superimposition region detection unit 23a detects a laparoscopic video beneath a superimposition region, which is a laparoscopic video obscured by the superimposed object. For example, in a case where only the laparoscopic video is input, the superimposition region detection unit 23a detects the laparoscopic video beneath the superimposition region using an AR marker or the like included in the laparoscopic video. In a case where the laparoscopic video and the surgical tool position information are input, in robotic surgery, the laparoscopic video beneath the superimposition region is detected by acquiring the position of the surgical tool from the medical device 12 without using the AR marker or the like. The surgical tool includes a tool for resecting an organ such as the liver. The detected laparoscopic video beneath the superimposition region is sent to the incident detection unit 23b. The incident detection unit 23b detects the change in the superimposition region and outputs the notification necessity information.

It is preferable that the incident detection unit 23b (incident detection unit 23c as well) be configured by a trained model that has been trained with learning data including a camera video (laparoscopic video) in which an incident has occurred. In addition, the notification necessity information may be differentiated for each incident as to whether notification is necessary or not, and the notification method may also be changed for each incident.

In addition, as shown in FIG. 7B, the detection unit 23 may be divided into two units of an incident detection unit 23c and a notification determination unit 23d. In this case, the laparoscopic video is input to the incident detection unit 23c. The incident detection unit 23c detects a region in which an incident has occurred in the laparoscopic video. The detection result is sent to the notification determination unit 23d together with the information on the superimposition region. The notification determination unit 23d outputs the notification necessity information based on whether or not the region in which an incident has occurred overlaps with the superimposition region in which the video of the superimposition target is superimposed on the laparoscopic video. In this case, in a case where the region in which the incident has occurred overlaps with the superimposition region, the notification necessity information indicating that notification is necessary is output.

Specifically, the detection unit 23 detects an incident as follows. The detection unit 23 detects the change in the pixel value of the superimposition region. Specifically, in a case where a differential pixel value of the superimposition region calculated from previous and next frames of the laparoscopic video falls outside a certain range, it is detected that there is a region in which an incident such as bleeding or bile leakage has occurred in the superimposition region.

In addition, the detection unit 23 detects outflow of a body fluid in the superimposition region. Specifically, an incident in which outflow of a body fluid occurs in the abdominal cavity includes bleeding and bile leakage. Therefore, in a case where at least one of bleeding or bile leakage is detected based on the outflow of the body fluid, the detection unit 23 outputs the notification necessity information indicating that notification is necessary. The outflow of the body fluid may be detected using a learning model, or may be detected based on pixel information obtained using a hyperspectral camera.

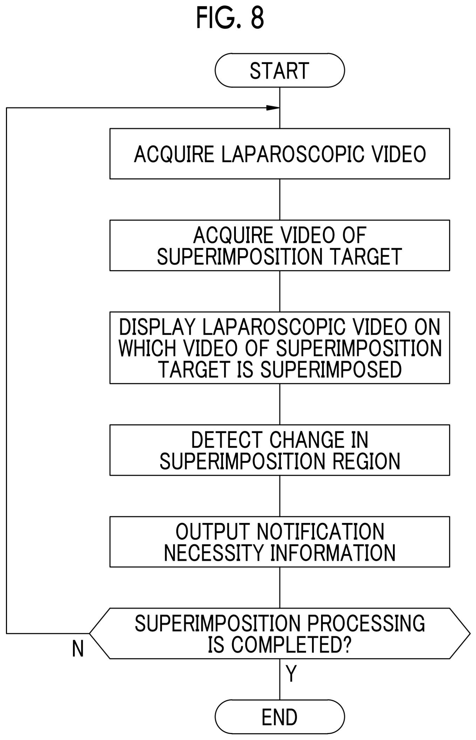

Next, a series of flows of outputting the notification necessity information according to the result of the change in the superimposition target will be described with reference to a flowchart of FIG. 8. The laparoscopic video 30 is acquired using the laparoscope 11. The video of the superimposition target is acquired from a server such as the PACS via the network NT. The display controller 22 causes the display 15 to display the laparoscopic video 30 on which the video of the superimposition target is superimposed. The detection unit 23 detects the change in the superimposition region in which the video of the superimposition target is superimposed, and outputs the notification necessity information according to the result of the detection. The series of flows described above is repeatedly performed until the superimposition processing of superimposing the video of the superimposition target is completed. It is preferable that the start and end of the processing relating to the superimposition of the superimposition region be controlled by user input, but this is not particularly limited.

In the present embodiment, each process is executed by any computer. In addition, any computer may execute the processing using a processor, a program, or a combination thereof. Any computer may be a general-purpose computer, a computer for a specific use, a system such as a workstation, or other hardware elements capable of executing a program.

The processor may be configured by one or more pieces of hardware, and the type of hardware is not limited. For example, the processor may be configured by a programmable logic device such as a central processing unit (CPU), a micro processing unit (MPU), or a field programmable gate array (FPGA), a dedicated circuit for executing specific processing such as an application specific integrated circuit (ASIC), or hardware such as a graphics processing unit (GPU) or a neural processing unit (NPU). In addition, the processor has each unit or each means that executes various types of processing in the present embodiment. In addition, the types of hardware may be a combination of different types of hardware. In a case where a plurality of pieces of hardware are configured to execute one or a plurality of processes of a certain processor, the plurality of pieces of hardware may be present in devices physically separated from each other, or may be present in the same device. In addition, in any of the embodiments, the order of each processing executed by the processor is not limited to the above order and may be changed as appropriate. The hardware is configured by an electric circuit (circuitry) in which circuit elements such as semiconductor elements are combined.

Further, the present embodiment may be realized by hardware, software, firmware, microcode, or a combination thereof. Software, firmware, and microcode are configured by a program. In addition, the program may be, for example, a program module group, and each function thereof may be realized by a processor configured to execute each function. The program may be a program code or a plurality of code segments stored in one or a plurality of non-transitory computer-readable media (for example, a recording medium or other storage). The program may be divided and stored in a plurality of non-transitory computer-readable media present in devices physically separated from each other. The program code or the code segment may represent any combination of a procedure, a function, a subprogram, a routine, a subroutine, a module, a software package, a class, an instruction, a data structure, or a program statement. The program code or the code segment may be connected to another code segment or a hardware circuit by transmitting and receiving information, data, an argument, a parameter, or memory contents.

### Explanation of References

10: medical system
11: laparoscope
12: medical device
14: medical image processing device
15: display
15a: main screen
15b: sub-screen
16: user interface
20: camera video acquisition unit
21: video acquisition unit for superimposition
22: display controller
23: detection unit
23a: superimposition region detection unit
23b: incident detection unit
23c: incident detection unit
23d: notification determination unit
30: laparoscopic video
31: liver
32: ultrasound probe
33: ultrasound image
35: vascular structure
P: patient
NT: network
OV: video of superimposition target

## Claims

1. A medical device (12) comprising:
a processor,
wherein the processor
acquires a camera video,
acquires a video of a superimposition target (OV) to be superimposed on the camera video, and
detects a change in a superimposition region in which the video of the superimposition target (OV) is superimposed on the camera video, and outputs any of notification necessity information according to a result of the detection, a superimposition parameter for controlling display of the superimposition region, or an incident occurrence region.

2. The medical device (12) according to claim 1, further comprising:
a display (15) configured to display a main screen (15a), or both a main screen (15a) and a sub-screen (15b),
wherein, in a case where the processor outputs the notification necessity information, in a case where the notification necessity information indicates that notification is necessary, the processor performs a two-screen display of the main screen (15a) and the sub-screen (15b), and, in a case where the notification necessity information indicates that notification is unnecessary, the processor performs a single-screen display of the main screen (15a).

3. The medical device (10) according to claim 1,
wherein the processor performs either increasing the transparency of the superimposition region or temporarily cancelling superimposition of the superimposition region by increasing or decreasing the superimposition parameter according to the result of the detection.

4. The medical device (12) according to claim 1,
wherein, in a case where the processor outputs the incident occurrence region, the processor performs either increasing the transparency of the incident occurrence region of the superimposition region or temporarily cancelling superimposition of the incident occurrence region.

5. The medical device (12) according to any one of claims 1 to 4,
wherein the processor detects a change in a pixel value of the superimposition region.

6. The medical device (12) according to any one of claims 1 to 4,
wherein the processor detects an outflow of a body fluid in the superimposition region.

7. The medical device (10) according to claim 5,
wherein, in a case where the processor detects at least one of bleeding or bile leakage based on the change in the pixel value, the processor outputs the notification necessity information indicating that notification is necessary.

8. The medical device (12) according to claim 6,
wherein, in a case where the processor detects at least one of bleeding or bile leakage based on a detection result of the outflow of the body fluid, the processor outputs the notification necessity information indicating that notification is necessary.

9. The medical device (12) according to any one of claims 1 to 4,
wherein the video of the superimposition target (OV) is at least one of an ultrasound video or a video of an anatomical structure or a lesion in preoperative 3D data.

10. The medical device (12) according to any one of claims 1 to 4,
wherein the superimposition target is at least one of a liver (31), a kidney, a spleen, a pancreas, a uterus, a nerve, a lung, a bronchus, an intracranial blood vessel, a prostate, or an organ that emits light due to a fluorescent dye.

11. An operation method of a medical device (12) including a processor, the operation method comprising:
a step of, via the processor, acquiring a camera video;
a step of, via the processor, acquiring a video of a superimposition target (OV) to be superimposed on the camera video; and
a step of, via the processor, detecting a change in a superimposition region in which the video of the superimposition target (OV) is superimposed on the camera video, and outputting any of notification necessity information according to a result of the detection, a superimposition parameter for controlling display (15) of the superimposition region, or an incident occurrence region.
